# FASCICULE DE BREVET EUROPEEN

(11) **EP 0 927 306 B1**
(45) Date de publication et mention de la délivrance du brevet: **23.01.2002**
(21) Numéro de dépôt: 97937395.8
(22) Date de dépôt: 08.09.1997
(51) Int. Cl.: F04B 43/12, A61M 5/142

(54) **POMPE PERISTALTIQUE PORTABLE**
TRAGBARE PERISTALTISCHE PUMPE
PORTABLE PERISTALTIC PUMP

(30) Priorité: 10.09.1996 FR 9611170
(43) Date de publication de la demande: 07.07.1999
(73) Titulaire: Precimedix S.A., 2000 Neuchâtel (CH)
(72) Inventeur: RAY, Claude, CH-2205 Montezillon (CH); TAILLARD, Christian, F-25500 Les Fins (FR)
(74) Mandataire: Gresset, Jean
(86) Numéro de dépôt international: CH9700327
(87) Numéro de publication internationale: WO9811349

(56) Documents cités:
- EP-A- 0 388 787
- EP-A- 0 521 184
- DE-C- 4 037 797
- US-A- 3 700 361
- US-A- 4 487 558
- PATENT ABSTRACTS OF JAPAN vol. 007, no. 163 (M-229), 16 juillet 1983 & JP 58 070079 A (PILOT PEN KK), 26 avril 1983,

## Description

La présente invention se rapporte aux pompes péristaltiques portables. Elle concerne, plus particulièrement, une pompe péristaltique miniature pour l'injection de solutions médicamenteuses.

Les pompes miniatures ou micropompes à usage médical sont connues depuis plusieures années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit pour autant alité et relié à un appareil encombrant, coûteux et bruyant.

De telles pompes sont, le plus souvent, de type péristaltique rotatif dont le principe consiste à disposer d'un tuyau en matière plastique déformable relié à un réservoir contenant la solution et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et refoulé vers la sortie pour être injecté dans le patient.

Les brevets EP 388 787, EP 447 909 , EP 521 184 et WO 94/06491 décrivent des pompes péristaltiques miniatures de ce type.

Lors de la conception de telles pompes, il est particulièrement important de se préoccuper du problème que pose la purge des tubulures, c'est à dire l'évacuation de l'air qu'elles contiennent encore, avant l'introduction de l'aiguille dans le patient. La vitesse de rotation du rotor étant très faible, généralement inférieure à 1 tour/minute, cette opération doit, pour ne pas prendre trop de temps, être effectuée en faisant tourner rapidement le rotor par des moyens externes. Dans le brevet EP 388 787, il est prévu une roue qui est solidaire du rotor et comporte une série de trous dans lesquels on peut introduire la pointe d'un stylo, par exemple, pour faire tourner la roue rapidement. La solution est, certes, intéressante mais aucune disposition n'est prévue pour éviter que cette opération n'endommage le train d'engrenages.

Le document JP 58 070079 décrit, par ailleurs, une pompe péristaltique dotée d'un système de débrayage permettant de désaccoupler le moteur et la tête de pompe afin de la faire tourner plus rapidement par une action manuelle. Il faut cependent noter que le débrayage se fait par actionnement en translation d'une tige de commande, ce qui rend l'opération peu pratique.

Un autre problème important qui se pose lors de la conception d'une pompe péristaltique miniature est celui du couplage entre les galets et le tuyau qu'ils viennent écraser contre la pièce d'appui.

Des mesures ont montré que la pression minimale sur un galet de 5mm de diamêtre nécessaire pour faire écouler un liquide dans un tuyau plastique ayant un diamètre interne de 1.47 mm et un diamètre externe de 1.96 mm est de 95 gr. La force de traction correspondante, exercée par le galet, est de 15 gr. La pression sur le galet peut monter jusqu'à 150 gr sans faire augmenter proportionnellement la force de traction qui passe alors seulement de 15 à 20 gr. Mais au delà de cette limite, la force de traction augmente très rapidement. Elle passe, en effet, à 50 gr pour une pression de 200 gr, après quoi les mesures deviennent impossibles.

De telles constatations s'expliquent aisément par le fait qu'une fois le tuyau comprimé jusqu'à sa complète obturation, toute augmentation de pression provoque la déformation du matériau plastique et la force de traction correspondante augmente alors en relation avec son module d'élasticité.

Ainsi, toute variation de position du galet par rapport au tuyau au delà de celle qui réalise son obturation sollicite fortement le rotor porte-galets et son moteur, ce qui provoque rapidement son bloquage et donc l'arrêt de la pompe. L'effet est d'autant plus marqué dans les pompes miniatures qui sont équipées de moteurs évidemment moins puissants que les pompes non portables.

A l'inverse, toute variation de position du galet en deçà de celle qui réalise la complète obturation du tuyau ne permet pas un écoulement normal du liquide.

Il est donc très important, pour obtenir une pompe à fonctionnement sûr, que la ditance séparant le galet de la pièce d'appui soit parfaitement définie et constante afin d'éviter le bloquage pur et simple ou un débit insuffisant.

Si donc la pièce d'appui et le galet sont fixes, des tolérances de fabrication extrêmement sévères sont nécessaires et augmentent ainsi très sensiblement le prix de revient de la pompe.

Il est donc préférable d'accepter des tolérances plus légères et de prévoir des moyens permettant de compenser automatiquement tout écart de position entre la pièce d'appui et le galet.

Les brevets EP 388 787 et EP 447 909 déjà cités décrivent succintement des arrangements pour résoudre ce problème.

Le brevet EP 388 787 montre une pièce d'appui présentant la forme d'un crochet articulé à l'une de ses extrémités par une goupille et pressé contre le tuyau à l'aide d'un ressort comprimé par une vis. Cette pièce d'appui étant pratiquement toujours sollicitée par deux galets, elle ne peut assurer la compensation des écarts de position pour chaque galet séparément.

Le brevet EP 477 909 montre que les galets sont montés sur leur axe avec un léger jeu radial leur permettant un certain débattement radial et que des ressorts à lame individuels, agissant directement sur leur partie centrale, les sollicitent vers l'extérieur. Une telle disposition présente le double inconvénient de compliquer le montage du rotor et de l'amener à glisser sur le tuyau plutôt que de tourner. Il est aussi indiqué, dans ce document, que les ressorts peuvent être remplacés par une pièce élastique unique, dont aucune description n'est cependant fournie et qu'ils peuvent être carrément omis car les galets sont alors déplacés radialement par l'élasticité inhérente du tuyau lui-même. Ceci montre que l'importance du problème n'a pas été pleinement perçue.

Les pompes dites à cassette sont les plus courantes dans les applications médicales. Elles comportent deux parties, d'une part, la pompe proprement dite, avec le moteur, l'électronique de pilotage, la pile et la tête de pompe formée du rotor et des galets presseurs et, d'autre part, la cassette qui s'enclipse sur la pompe et comprend le tuyau et la pièce d'appui. Le réservoir est soit intégré dans la cassette lorsqu'il est de petites dimensions, soit disposé librement à l'extérieur lorsqu'il est de grandes dimensions.

Les pompes des brevets EP 447 909, EP 521 184 et WO 94/06491 sont de ce type.

Dans les trois cas, la cassette comporte, assemblés de manière définitive, le tuyau, la pièce d'appui et le réservoir. Les pompes des deux brevets EP ne sont utilisables qu'une seule fois car, une fois accouplés, les deux modules ne peuvent plus être désaccouplés. A la fin du traitement ou lorsque la cassette est vide, on jette le tout, y compris donc le moteur, la tête de pompe, la pièce d'appui, le train d'engrenages et le circuit qui pourtant sont tous capables de fonctionner à nouveau. Quant à la pompe du brevet WO, sa cassette est jettable en même temps que son réservoir. Ainsi, dans ces trois pompes, on jette des composants qui pourraient encore être utilisés tels quels puisqu'ils ne sont pas usés et n'ont jamais été en contact avec le médicament injecté dans le malade.

Les documents US 4 817 057 et EP 120 284 décrivent, par ailleurs, des pompes péristaltiques à cassette de grandes dimensions, c'est à dire non portables. Bien que le tuyau puisse être aisément inséré dans - ou enlevé de - la cassette, il est muni d'un embout de raccordement à la tubulure provenant du réservoir et sa portion soumise à l'action péristaltique est spécialement conçue pour résister à une exposition prolongée à l'effet de compression des galets. Une telle construction présente des inconvénients car, non seulement, elle n'interdit pas la réutilisation du tuyau pour administrer des solutions médicamenteuses différentes à des patients différents, mais encore, les opérations d'interconnexion des deux tubes accroissent le risque de pollution du circuit par des bactéries.

La présente invention a pour but de fournir une pompe péristaltique portable qui est exempte des inconvénients des pompes de l'art antérieur.

La pompe selon l'invention comporte:
- un rotor équipé d'au moins un galet tournant,
- des moyens d'entraînement dudit rotor,
- des moyens de commande desdits moyens d'entraînement,
- une pièce d'appui munie d'une portion arrondie disposée de manière sensiblement concentrique audit rotor et contre laquelle, en fonctionnement, ledit galet vient comprimer un tuyau souple relié à un réservoir de liquide pour pousser celui-ci vers l'extérieur et
- des moyens pour faire tourner ledit rotor par une action externe afin de réaliser la purge du tuyau.

Cette pompe est principalement caractérisée en ce que le rotor est réalisé en deux parties concentriques superposées dont l'une est couplée auxdits moyens d'entraînement et dont l'autre porte ledit galet et en ce qu'il comporte, en outre, des moyens d'embrayage-débrayage grâce auxquels:
- en fonctionnement normal, ses deux parties coopèrent pour réaliser sa mise en rotation par ses moyens d'entraînement,
- lorsqu'il est mis en rotation par une action externe, lesdites parties sont automatiquement désaccouplées.

D'autres caractéristiques de la présente invention ressortiront de la description qui va suivre, faite en regard des dessins annexés et donnant, à titre explicatif mais nullement limitatif, une forme préférée de réalisation de cette pompe. Sur ces dessins:
- la figure 1 est vue générale extérieure du module pompe;
- la figure 2 représente le module réservoir;
- la figure 3 représente le module cassette;
- la figure 4 montre la façon dont les trois modules sont accouplés;
- la figure 5 est un détail en coupe de la figure 4 lorsque l'accouplement est réalisé;
- la figure 6 est une vue générale de la pompe assemblée;
- la figure 7 est une vue de dessus du module pompe;
- la figure 8 est une vue en coupe du module pompe selon la ligne VIII-VIII de la figure 7;
- la figure 9 est une vue de dessus du rotor du module pompe;
- la figure 10 montre le module électronique de commande de la pompe;
- la figure 11 est un schéma du circuit de commande de la pompe:
- enfin, la figure 12 illustre une variante de réalisation du module réservoir.

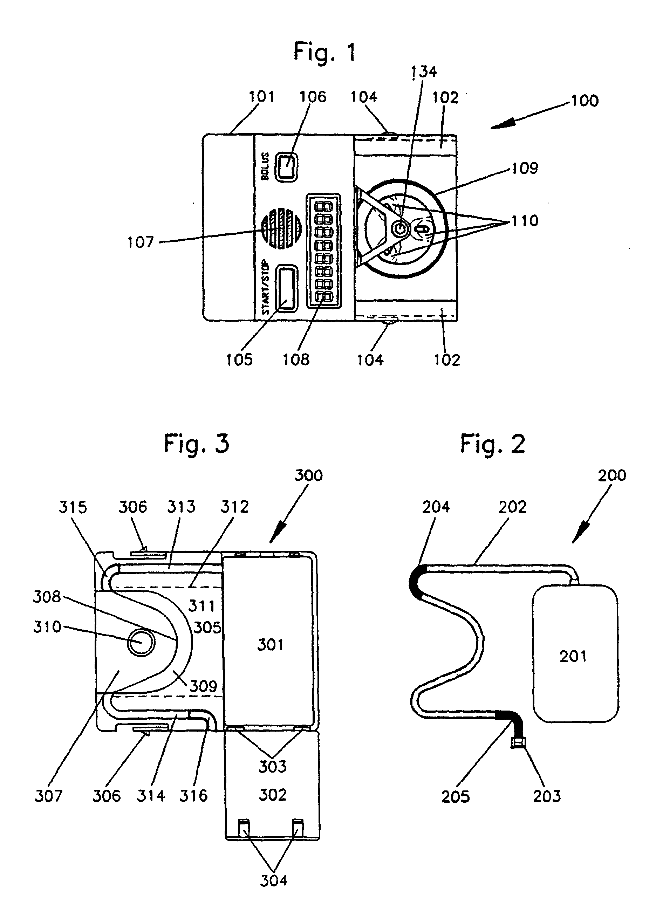

Ainsi que le montrent les figures 1 à 5, la pompe miniature selon l'invention est constituée de trois modules, un module pompe 100, un module réservoir 200 et un module cassette 300 qui seront aussi appelés respectivement pompe, réservoir et cassette. Le réservoir 200 se place de façon amovible dans la cassette 300 qui, à son tour, s'accouple de façon amovible à la pompe 100.

Pour fixer les idées et à simple titre d'exemple, la pompe assemblée a une longueur de 110 mm, une largeur de 55 mm et une épaisseur de 13 mm pour une capacité de réservoir de 10 ml.

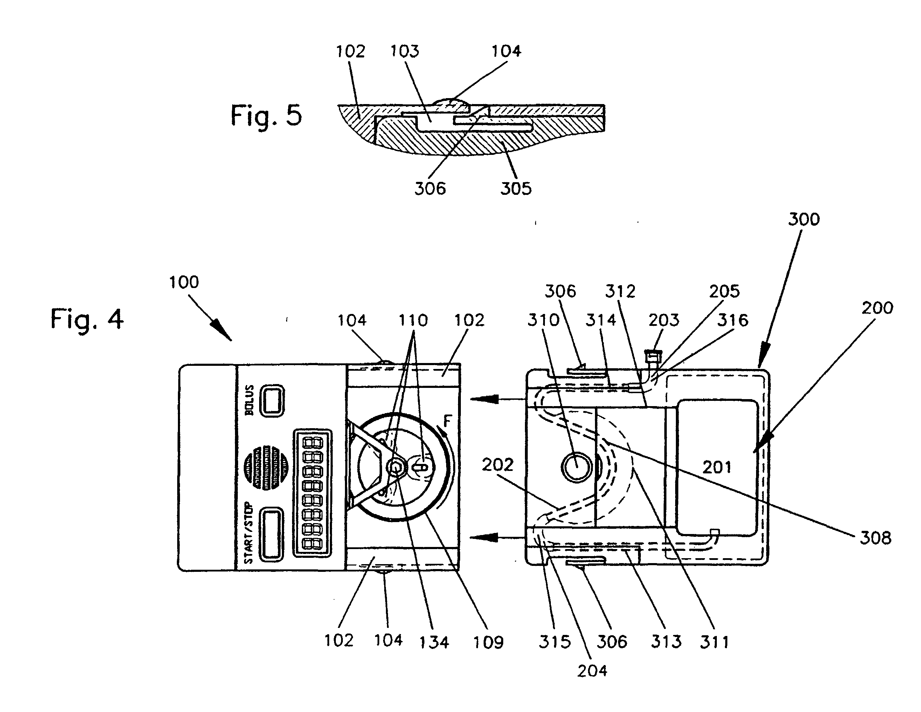

Le module pompe 100, représenté sur la figure 1, comporte un boîtier en plastique rigide 101 dont le fond se prolonge d'un côté pour former la base de deux coulisses parallèles 102 servant à la mise en place du module cassette 300 à la manière d'un tiroir. Ainsi qu'il apparaît mieux sur la figure 5, chaque coulisse 102 est percée d'une ouverture 103 dans laquelle est ménagée une languette souple 104 formant un bouton poussoir destiné au dégagement de la cassette lorsqu'elle doit être désaccouplée de la pompe.

Sur sa face supérieure, le boîtier 101 comporte un bouton "START/STOP" 105 servant à commander le démarrage et l'arrêt de la pompe, un bouton "BOLUS" 106 servant à déclencher l'administration de doses additionnelles de solution, un avertisseur acoustique 107 et un affichage LCD 108.

Le boîtier 101 laisse apparaître, entre ses deux coulisses 102, un rotor 109 portant trois galets 110 et formant la tête de la pompe. Une description détaillée en sera donnée plus loin en même temps que celle de ses moyens d'entraînement.

Le module réservoir 200, représenté sur la figure 2, est formé d'une poche en plastique 201 ayant, dans le mode de réalisation représenté, un volume de 10 ml et d'un tuyau en plastique souple 202 dont une extrémité est reliée à la poche et dont l'autre, destinée à être connectée à une aiguille d'injection sous-cutanée ou intraveineuse, est obturée par un bouchon 203. Le tuyau comporte, en outre, en surépaisseur, deux raccords moulés rigides en forme de coude 204 et 205 servant à son enclipsage dans la cassette 300, ainsi qu'il apparaîtra mieux plus tard.

Le module cassette 300, représenté vu de dessous sur la figure 3, est réalisé en plastique rigide et comporte un logement 301 destiné à recevoir la poche plastique 201. Il a sensiblement la même épaisseur et la même largeur que le boîtier 101 et possède, côté fond, un couvercle 302 monté sur deux charnières 303 et muni, à l'autre extrémité, de languettes 304 assurant sa fermeture par enclipsage. Le logement 301 se prolonge par un plateau 305 profilé et dimensionné pour prendre place, comme un tiroir, entre les coulisses 102 du boîtier. Deux languettes 306, ménagées sur ses côtés, y permettent son enclipsage par arrimage de leur extrémité dans les ouvertures 103, ainsi que le montre également la figure 5.

Le plateau 305 est percé, côté fond, d'un logement 307, en forme générale de U, dimensionné de manière à recevoir le rotor 109 du module pompe. Il comporte, à cet effet, une partie centrale arrondie 308 de 120 degrés environ et dont le rayon est légèrement supérieur à celui du cercle que parcourt la face externe des galets 110. C'est sur cette portion arrondie 308 qu'en fonctionnement, les trois galets viennent tour à tour écraser le tuyau flexible 202. Un dégagement supplémentaire arrondi 309 est prévu autour de la portion 308 pour recevoir la partie inférieure du rotor 109, de plus grand diamètre que le cercle parcouru par les galets, comme il apparaîtra plus loin.

Le fond du logement 307, qui forme la paroi supérieure du module, est percé, au centre de la portion arrondie 308, d'un orifice circulaire 310 au regard de l'endroit où prend place l'axe du rotor 109 afin de permettre la purge de la pompe avant usage, comme il sera décrit plus loin. Cet orifice est obturable par un couvercle 311 coulissant dans un évidement 312 de la paroi supérieure du module.

Le plateau 305 comporte également deux canaux 313 et 314 ménagés chacun dans le fond d'un de ses côtés parallèlement à celui-ci. Le canal 313 relie le logement 301 à la partie antérieure du logement 307. Avant de déboucher dans celle-ci, il présente un portion coudée 315, de plus grand diamêtre, formée et dimensionnée de manière à recevoir et maintenir par enclipsage le raccord rigide 204 du tuyau souple 202. Le canal 314 part de la partie antérieure du logement 307 à l'opposé de l'endroit où arrive l'autre canal et débouche à l'extérieur après une portion coudée 316, de plus grand diamêtre, formée et dimensionnée de manière à recevoir et maintenir par enclipsage le raccord rigide 205 du tuyau 202.

On se référera maintenant plus spécialement aux figures 4, 5 et 6 qui montrent la façon dont les trois modules précédemment décrits s'accouplent pour former la pompe miniature selon l'invention.

Le module réservoir 200 est, tout d'abord, mis en place dans le module cassette 300. La poche plastique 201 est introduite dans son logement 301 et le tuyau souple 202 positionné dans les canaux 313 et 314 en veillant à ce que ses deux raccords rigides 204 et 205 prennent place et s'enclipsent respectivement dans les portions coudées 315 et 316 du plateau 305. Après avoir refermé le couvercle 302, il ne reste plus alors qu'à insérer la cassette dans les coulisses 102 du module pompe 100 jusqu'à ce que les languettes 306 s'accrochent dans les ouvertures 103 (figure 5). L'extrémité de la partie inférieure du rotor 109 vient occuper le dégagement 309 du plateau 305, tandis que la portion centrale du tuyau souple 202 se trouve automatiquement emprisonnée entre le rotor 109 et la pièce d'appui 308. Lorsque le rotor sera mis en rotation dans le sens de la flèche F (sens inverse des aiguilles d'une montre), les galets 110 viendront tour à tour comprimer le tuyau 102 pour pousser vers l'extérieur la solution qu'il contient.

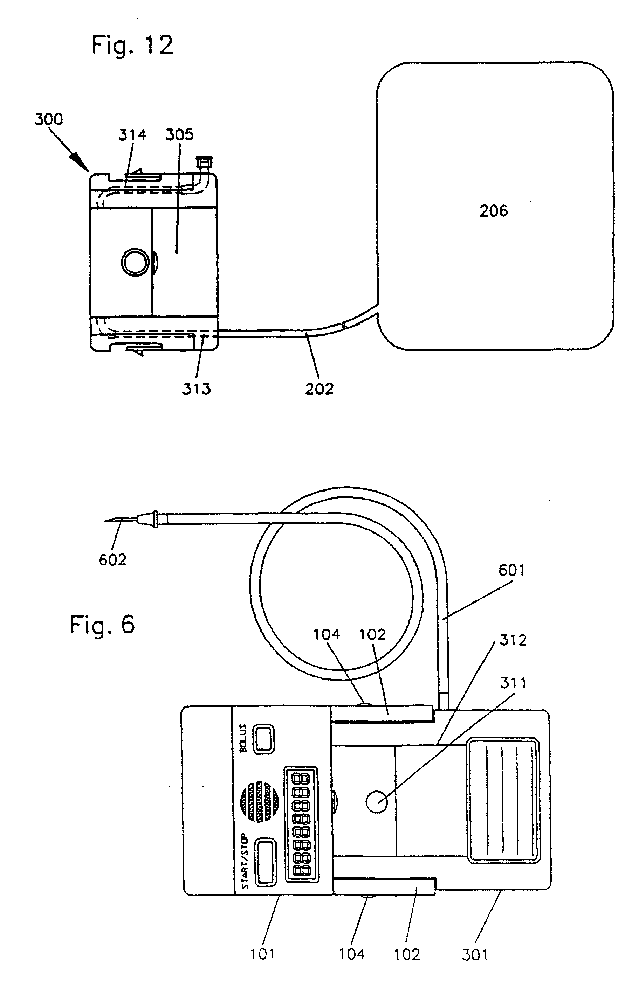

La pompe étant ainsi chargée, le bouchon 203 du tuyau est enlevé et remplacé, comme le montre la figure 6, par un tuyau 601 se terminant par une aiguille 602. Avant d'introduire cette dernière dans le patient, il est impératif d'évacuer l'air présent dans le circuit et d'y faire arriver la solution à injecter. La vitesse de rotation normale du rotor 109 étant très faible (0.625 tour/minute). ainsi qu'il sera décrit plus loin, cette opération de purge doit, pour ne pas prendre trop de temps, être effectuée en faisant tourner rapidement le rotor par des moyens externes. L'extrémité supérieure de l'axe de ce dernier présente, à cet effet, une ouverture de forme 134 apparaîssant dans l'ouverture 310 de la cassette et qui permet, à l'aide d'un outil approprié, la rotation rapide du rotor jusqu'à ce que des gouttes de la solution à injecter sortent de l'aiguille 602. Celle-ci peut être alors introduite dans le patient, après quoi la pompe est mise en marche en appuyant sur le bouton 105 qui commande la mise en rotation du rotor 109 par des moyens qui seront décrits plus loin en détail.

En fin de traitement, le désaccouplement des modules pompe 100 et cassette 300 est réalisé en appuyant sur les deux boutons 104 de manière à dégager les languettes 306 des ouvertures 103. La cassette peut alors être extraite en la glissant vers l'extérieur avant d'en retirer le réservoir 200 qui peut être remplacé par un autre, la cassette et la pompe pouvant être réutilisées pour un autre traitement.

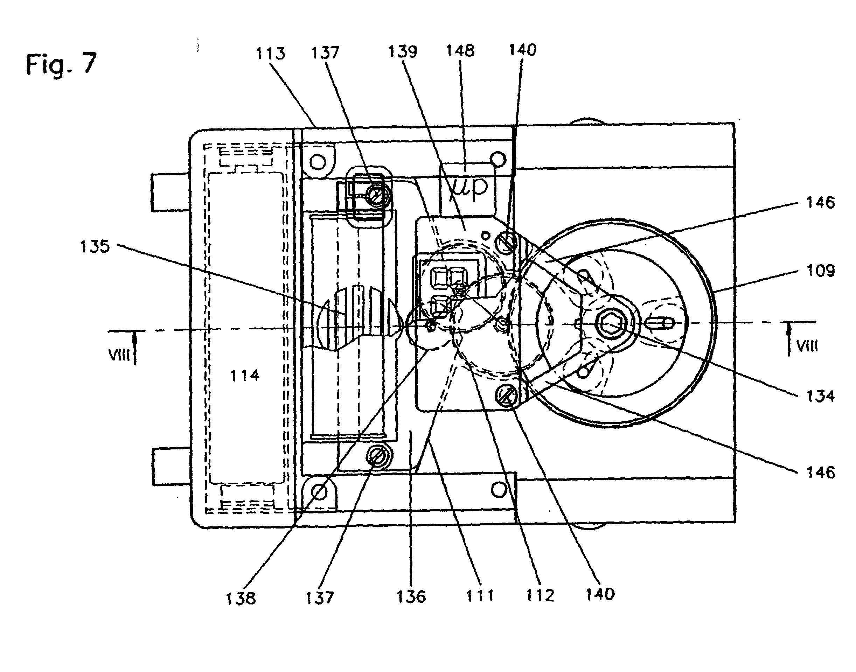

Le module pompe 100 va être maintenant décrit en se référant aux figures 7 à 11 qui montrent le rotor 109, un moteur pas à pas 111, un train d'engrenages 112 couplant le moteur au rotor, un module électronique de commande 113 et une pile bâton 114.

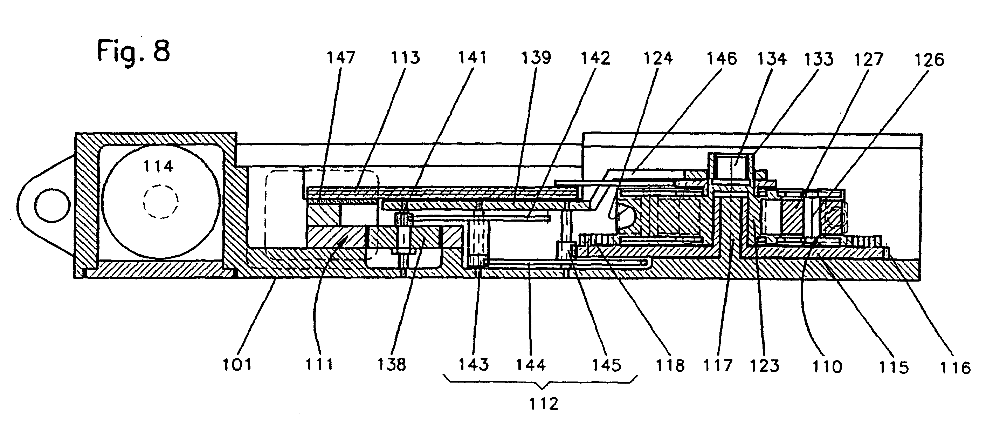

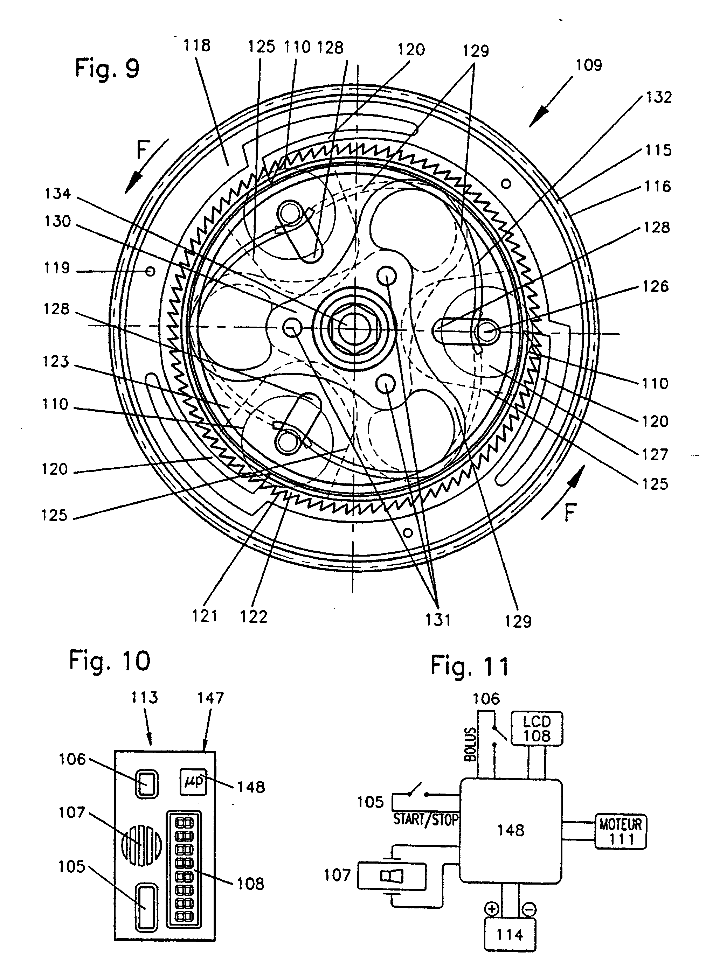

Le rotor 109, représenté en détail sur les figures 8 et 9, comporte une base circulaire 115 munie d'une denture périphérique 116 et montée en libre rotation sur un pivot 117 faisant partie du fond du boitier 101. Une couronne 118, de diamêtre extérieur légèrement inférieur à celui de la base 115, est montée sur cette dernière et fixée à elle par des vis 119. Cette couronne présente, vers l'intérieur, trois bras souples 120 découpés dans sa masse à 120° l'un de l'autre et formant des cliquets dont l'extrémité coopère avec la périphérie en dents de scie 121 d'une roue 122. Celle-ci constitue la base d'un plateau porte-galets cylindrique 123 monté sur l'axe de la base 115 autour duquel il peut tourner librement.

La paroi périphérique du plateau 123 a un profil incurvé vers l'intérieur 124 de manière à former un canal pour le passage du tuyau souple 202. Cette même paroi est également percée de trois logements 125 en forme de U disposés à 120° l'un de l'autre pour recevoir les trois galets 110 de compression du tuyau.

Chaque galet 110 est formé d'un axe 126 et d'un corps cylindrique 127 monté sur l'axe autour duquel il peut tourner librement. Les deux extrémités de cet axe prennent place dans des ouvertures oblongues 128 ménagées radialement dans les portions du plateau 123 formant les parois planes inférieure et supérieure des logements 125. Les galets sont maintenus en position verticale, c'est à dire avec leur axe parallèle à l'axe du rotor, et soumis à une force radiale dirigée vers l'extérieur grâce à deux ressorts 129 disposés de part et d'autre du plateau 123. Chaque ressort comporte une partie centrale rigide 130, de forme sensiblement triangulaire, fixée au plateau concentriquement à lui par des rivets 131 et trois bras ressorts flexibles recourbés 132 dont les extrémités libres prennent appui sur les extrémités respectives des axes 126 et les poussent vers l'extérieur de manière à ce que les galets 110 exercent sur le tuyau 202 une force sensiblement constante, fixée à 120 grammes dans l'exemple décrit. Les erreurs de positionnement relatif des galets et du tuyau, dues aux inévitables défauts de fabrication, sont ainsi automatiquement compensées, ce qui évite une compression excessive ou insuffisante du tuyau 202.

Le plateau 123 se prolonge axialement vers la haut par une protubérance 133 traversant la partie centrale 130 du ressort supérieur 129. Cette protubérance est percée de l'ouverture de forme 134 déjà citée dans laquelle peut s'introduire, à travers l'orifice circulaire 310 de la cassette, la pointe profilée de manière correspondante d'un outil (non représenté) permettant la rotation rapide du plateau pour effectuer la purge de la pompe.

L'entraînement en rotation du rotor 109 est assuré par le moteur pas à pas 111 qui est du type monophasé bipolaire classique et tourne à la vitesse de 16 tours/seconde à raison de deux pas par tour. Son noyau-bobine et son stator, respectivement désignés par les références 135 et 136, sont fixés sur des pieds formés dans le fond du boîtier 101 par deux vis 137. Son rotor 138 pivote entre le fond du boîtier et un pont 139 fixé par des vis 140 sur des pieds solidaires du fond du boîtier.

Le rotor 138 porte un pignon 141 engrènant avec une roue 142 formant le premier mobile du train d'engrenages 112. Le pignon 143 de cette roue engrène lui-même avec une roue 144 dont le pignon 145 engrène enfin avec la denture périphérique 116 de la base 115 du rotor 109 pour la faire tourner à la vitesse de 0.625 tour par minute. Les roues 142 et 144 pivotent, comme le rotor 138, entre le fond du boîtier et le pont 139. Ce dernier se termine, après un coude, par deux bras 146, non représentés sur la figure 9, dont l'extrémité commune comporte une ouverture circulaire dans laquelle est maintenue et pivote la protubérance 133 du rotor 109.

En fonctionnement, lorsque la base 115 du rotor est entraînée dans le sens de la flèche F par le moteur 111 par l'intermédiaire du train d'engrenages 112, elle fait tourner avec elle la couronne 118 dont elle est solidaire. Les trois cliquets 120 de cette dernière coopèrent alors avec la périphérie en dents de scie 121 de la roue 122 pour la mettre en rotation et, avec elle, le plateau porte-galets 123 dont elle forme la base. Le galets 110 viennent ainsi tour à tour comprimer le tuyau 102 pour pousser le liquide qu'il contient en direction de l'aiguille d'injection 602.

Lorsque, pour réaliser la purge des tubulures, on fait tourner le plateau porte-galets 123 à l'aide d'un outil de forme appropriée introduit dans l'ouverture 134, la base 115 et la couronne 118 deviennent fixes, car freinées par le couple de rétention du moteur 111 et son train d'engrenages 112. Un débrayage s'opère alors automatiquement entre la couronne 118 et la roue 122 du fait que la rotation de celle-ci provoque l'expulsion des trois cliquets 120 des dents de scie 121. Le plateau porte-galets 123 peut ainsi être tourné rapidement sans effet sur le train d'engrenages et le moteur.

Le module électronique de commande 113, représenté sur la figure 10, a pour base une plaquette de circuit imprimé 147 fixée sous la face supérieure du boîtier et portant les boutons 105 et 106, l'avertisseur acoustique 107 et l'affichage LCD 108. La plaquette porte également un circuit microprocesseur 148 intégrant les principales fonctions suivantes : doubleur de tension, base de temps à quartz, mémoire, driver LCD et générateur de son. Ce microprocesseur étant d'un type connu, tel que le EPSON 62L35, il ne sera pas décrit en détail. L'ensemble de ces composants ainsi que la pile 114 et la bobine du moteur 111 sont interconnectés selon le schéma simplifié de la figure 11.

Le microprocesseur 148 est programmé, selon des moyens bien connus de l'homme de métier, pour que l'ensemble fonctionne comme suit.

Lorsque l'interrupteur 105 est actionné pour la première fois, il déclenche le fonctionnement du microprocesseur 148 qui, sous les ordres d'un programme d'administration enregistré dans la mémoire, applique aux bornes du moteur pas à pas 111 des impulsions motrices à la fréquence de 32 Hz provoquant sa rotation à la vitesse de 16 tours/sec. Le rotor porte-galets 109 est alors entraîné à la vitesse de 0.625 tour/min. Le fonctionnement de la pompe est interrompu lorsque l'interrupteur 105 est actionné une deuxième fois.

Pour fixer les idées, avec un rayon de rotation du plateau porte-galets de 10.44 mm et une vitesse de 0.625 tour/min, la solution progresse dans le tuyau à la vitesse de 41 mm/min. En choisissant un tuyau ayant un diamètre intérieur de 1.47 mm, le débit instantané de la pompe est ainsi de 69.54 mm3/min, soit 100 cm3/jour.

Le microprocesseur 148 permet également, en réponse à l'actionnement du bouton 106, d'échapper au programme d'administration mis en mémoire pour commander l'injection d'une quantité supplémentaire déterminée de solution (bolus). Cette procédure est plus particulièrement destinée aux traitements anti-douleur.

La pompe est également munie de moyens permettant d'actionner l'avertisseur acoustique 107 et/ou l'affichage LCD 108 en cas de mauvais fonctionnement : non respect du programme, arrêt, obstruction de l'aiguille, fin de vie de la pile....Ces moyens ne faisant pas l'objet du présent brevet, ils ne seront pas décrits.

On se référera enfin à la figure 12 qui montre une variante de réalisation du module cassette 300 pour des traitements nécessitant l'injection d'importantes quantités de solution. La cassette peu, certes, être rallongée pour recevoir un réservoir plus grand, mais si le volume de celui-ci dépasse 20 ml, l'encombrement de la pompe risque de la rendre peu pratique. Pour cette raison, le module cassette de la figure 12 ne comporte que le plateau 305 décrit à la figure 3, les mêmes références étant utilisées pour désigner les mêmes éléments. Le module réservoir comprend alors une poche de grand volume 206, qui n'est pas introduite dans de la cassette mais seulement reliée à elle par un tuyau souple 202 plus long identique à celui décrit à la figure 2.

## Revendications

1. Pompe péristaltique portable comportant:
- un rotor (109) équipé d'au moins un galet tournant (110),
- des moyens d'entraînement (111, 112) dudit rotor,
- des moyens de commande (113) desdits moyens d'entraînement,
- une pièce d'appui munie d'une portion arrondie (308) disposée de. manière sensiblement concentrique au rotor et contre laquelle, en fonctionnement, ledit galet vient comprimer un tuyau souple (202) relié à un réservoir de liquide (210) pour pousser celui-ci vers l'extérieur et
- des moyens pour faire tourner ledit rotor par une action externe afin de réaliser la purge du tuyau,
ladite pompe étant **caractérisée en ce que** le rotor est réalisé en deux parties concentriques superposées dont la première est couplée auxdits moyens d'entraînement et dont la deuxième porte ledit galet et **en ce qu'**il comporte, en outre, des moyens d'embrayage-débrayage grâce auxquels:
- en fonctionnement normal, ses deux parties coopèrent pour réaliser sa mise en rotation par ses moyens d'entraînement,
- lorsqu'il est mis en rotation par une action externe, lesdites parties sont automatiquement désaccouplées.

2. Pompe péristaltique portable selon la revendication 1, **caractérisée en ce que** la première partie du rotor comporte une roue dentée (115) coopérant avec lesdits moyens d'entraînement, **en ce que** sa deuxième partie comporte également une roue dentée (122) formant la base d'un plateau porte-galet (123) et **en ce que** lesdits moyens d'embrayage-débrayage comportent une couronne (118) fixée sur la roue dentée (115) de la première partie concentriquement à elle, entourant la roue dentée (122) de la deuxième partie et présentant, vers l'intérieur, au moins un bras souple (120) formant cliquet et dont l'extrémité coopère avec la roue dentée de la deuxième partie.

3. Pompe péristaltique portable selon l'une des revendications précédentes, **caractérisée en ce que** les moyens pour faire tourner le rotor par une action externe comportent une protubérance (133) prolongeant l'axe du rotor et percée d'une ouverture de forme (134) destinée à recevoir l'extrémité d'un outil de forme correspondante.

4. Pompe péristaltique portable selon l'une des revendications précédentes, **caractérisée en ce que**:
- le rotor et son galet, lesdits moyens d'entraînement et lesdits'moyens de commande forment un premier module (100), dit module pompe,
- la pièce d'appui fait partie d'un deuxième module (300), dit module cassette,
- lesdits modules sont dotés chacun de moyens permettant leur accouplement et
- le module cassette comporte des moyens (313, 314) pour recevoir de manière interchangeable ledit tuyau qui fait partie indissociable du réservoir et le positionner automatiquement de manière à ce qu'au moment de l'accouplement des deux modules, il s'applique contre ladite portion arrondie pour y être comprimé par ledit galet, le tuyau et le réservoir formant ainsi un troisième module (200), dit module réservoir, qui peut être aisément échangé avec un autre après usage.

5. Pompe péristaltique portable selon la revendication 4, **caractérisée en ce que** le module cassette comprend un plateau (305) s'engageant, comme un tiroir, dans le module pompe et percé d'un logement (307), en forme générale de U, profilé et dimensionné pour entourer le rotor et dont le fond arrondi (308) constitue la pièce d'appui du tuyau et **en ce que** les moyens pour recevoir et positionner ledit tuyau comportent un premier (313) et un deuxième (314) canal ménagés le long des côtés respectifs dudit plateau, lesdits canaux ayant une extrémité ouverte sur l'extérieur du plateau et leurs autres extrémités débouchant à l'opposé l'une de l'autre dans l'entrée dudit logement.

6. Pompe péristaltique portable selon la revendication 5, **caractérisée en ce que** lesdits canaux comportent des moyens pour retenir ledit tuyau par enclipsage.

7. Pompe péristaltique portable selon la revendication 6, **caractérisée en ce que** lesdits moyens pour retenir le tuyau sont constitués par des portions coudées (315, 316) desdits canaux, profilées et dimensionnées pour que des portions (204, 205) de forme correspondante dudit tuyau y prennent place et y soient retenues par enclipsage.

8. Pompe péristaltique portable selon l'une des revendications 4 à 7, **caractérisée en ce que** le module cassette se fixe dans le module pompe par enclipsage de son plateau dans des coulisses (102) dudit module pompe.

9. Pompe péristaltique portable selon l'une des revendications 4 à 8, **caractérisée en ce que** le module cassette comprend, en outre, un logement (301) pour recevoir le réservoir de solution.

10. Pompe péristaltique portable selon l'une des revendications précédentes, **caractérisée en ce que** le rotor comporte des moyens de compensation automatique des écarts de position entre le galet et la pièce d'appui.

11. Pompe péristaltique portable selon la revendication 10, **caractérisée en ce que** le galet (110) comporte un corps (127) sensiblement cylindrique et un axe (126) sur lequel ledit corps est monté rotatif, **en ce que** les extrémités dudit axe prennent place dans des ouvertures oblongues (128) ménagées radialement dans le rotor et **en ce que** lesdits moyens de compensation comportent deux ressorts (129) disposés au niveau des extrémités respectives de l'axe du galet et comprenant chacun une partie centrale (130) concentrique au rotor et un bras ressort recourbé (132) dont une extrémité est solidaire de ladite partie centrale et dont l'autre extrémité prend place contre l'extrémité correspondante de l'axe du galet pour le pousser radialement vers l'extérieur et permettre ainsi audit galet d'exercer sur le tuyau une force de compression sensiblement constante.

## Claims

1. Portable peristaltic pump comprising:
- a rotor (109) equipped with at least one rotating roller (110),
- means for driving (111, 112) said rotor,
- means for controlling (113) said driving means,
- a support element designed with a rounded portion (308) arranged substantially concentric to the rotor and against which, in operation, said roller compresses flexible tubing (202) connected to a liquid tank (201) to push it outwards, and
- means for rotating said rotor by external action in order to bleed the tubing,
said pump being **characterized in that** the rotor is designed in two superposed concentric parts the first of which is coupled to said driving means and the second of which carries said roller, and **in that** it comprises, in addition, clutching-declutching means thanks to which:
- in normal operation, its two parts cooperate to achieve its rotation by its driving means,
- when it is rotated by external action, said parts are automatically decoupled.

2. Portable peristaltic pump according to Claim 1, **characterized in that** the first part of the rotor comprises a gear wheel (115) cooperating with said driving means, **in that** its second part also comprises a gear wheel (122) forming the base of a roller-holding plate (123) and **in that** said clutching-declutching means comprise a crown wheel (118) fixed on the gear wheel (115) of the first part, concentric to it, surrounding the gear wheel (122) of the second part and presenting, inwards, at least one flexible arm (120) forming a pawl and whose end cooperates with the gear wheel of the second part.

3. Portable peristaltic pump according to any of the preceding claims, **characterized in that** the means for rotating the rotor by external action include a protuberance (133) extending the shaft of the rotor and pierced with an opening having a form (134) designed to receive the end of a tool of corresponding form.

4. Portable peristaltic pump according to any of the preceding claims, **characterized in that**:
- the rotor and its roller, said driving means and said control means form a first module (100), called the pump module,
- the supporting element forms part of a second module (300), called the cassette module,
- said modules are each provided with means enabling their coupling, and
- the cassette module has means (313, 314) for receiving interchangeably said tubing which forms an indissociable part of the tank and positioning it automatically so that, when the two modules are coupled, it is applied against said rounded portion to be compressed by said roller, the tubing and the tank thus forming a third module (200), called the tank module, which may be easily exchanged with another after use.

5. Portable peristaltic pump according to Claim 4, **characterized in that** the cassette module comprises a plate (305) fitting, like a drawer, in the pump module and pierced with a recess (307), in general U form, designed and dimensioned to surround the rotor and whose rounded bottom (308) constitutes the support element of the tubing and **in that** the means for receiving and positioning said tubing comprise a first (313) and a second (314) channel provided along the respective sides of said plate, said channels having one end open on to the outside of the plate and the other ends leading opposite each other to the entry of said recess.

6. Portable peristaltic pump according to Claim 5, **characterized in that** said channels comprise means for retaining said tubing by clipping.

7. Portable peristaltic pump according to Claim 6, **characterized in that** said means for retaining the tubing consist of elbowed portions (315, 316) of said channels, designed and dimensioned so that portions (204, 205) of corresponding form of said tubing fit there and are retained there by clipping.

8. Portable peristaltic pump according to any of Claims 4 through 7, **characterized in that** the cassette module is secured in the pump module by the clipping of its plate in slides (102) of said pump module.

9. Portable peristaltic pump according to any of Claims 4 through 8, **characterized in that** the cassette module comprises, in addition, a recess (301) receiving the solution tank.

10. Portable peristaltic pump according to any of the preceding claims, **characterized in that** the rotor comprises means for automatically compensating for position deviations between the roller and the support element.

11. Portable peristaltic pump according to Claim 10, **characterized in that** the roller (110) comprises a substantially cylindrical body (127) and a shaft (126) on which said body is mounted rotatingly, **in that** the ends of said shaft fit into oblong openings (128) provided radially in the rotor and **in that** said compensation means comprise two springs (129) placed at the respective ends of the roller shaft and each comprising a central part (130) concentric to the rotor and a curved spring arm (132) one end of which is integral with said central part and the other end of which is placed against the corresponding end of the roller shaft to push it radially outwards and thus allow said roller to exert a substantially constant compression force on the tubing.

## Patentansprüche

1. Tragbare Peristaltikpumpe mit:
- Einem Rotor (109) mit mindestens einer drehbaren Rolle (110),
- Vorrichtungen zum Antrieb des Rotors (111, 112),
- Steuerungen (113) für die Antriebsvorrichtungen,
- Einem Stützstück mit einem abgerundeten Teil (308). Dieser ist konzentrisch zum Rotor angebracht, bei Betrieb wird gegen diesen von den Rollen ein flexibler Schlauch (202) komprimiert,. der mit einem Flüssigkeitstank verbunden ist (201). So wird der abgerundete Anteil nach außen gepresst.
- Vorrichtungen zur Drehung des Rotors über externe Befehle, um den Schlauch zu entlüften.
Bei dieser Pumpe besteht der Rotor aus zwei konzentrischen Teilen, die übereinander angebracht sind. Der erste Teil ist an die Antriebsvorrichtungen gekoppelt und der zweite Teil trägt die Rolle. Des Weiteren verfügt er über eine Ein- und Ausrückvorrichtung mit der:
- Bei gewöhnlichem Betrieb diese beiden Teile zusammenwirken, um ihn über die Antriebsvorrichtung zu drehen.
- Die beiden Teile automatisch entkoppelt werden, wenn er über einen externen Befehl in Rotation versetzt wird.

2. Tragbare Peristaltikpumpe nach Anspruch 1, bei der der erste Teil des Rotors mit einem Zahnrad (115) ausgestattet ist, das mit der Antriebsvorrichtung gekoppelt ist. Auch der zweite Teil ist mit einem Zahnrad ausgestattet (122), das die Funktion eines Trägergestells für eine Rollen-Trägerplatte (123) übernimmt. Die Ein- und Ausrückvorrichtung ist mit einem Radkranz (118) ausgeführt, der auf dem Zahnrad (115) des ersten konzentrischen Teils befestigt ist und das Zahnrad des zweiten Teils (122) umgibt. Nach innen bildet so mindestens ein flexibler Arm (120) einen Verschluss, dessen Ende mit dem Zahnrad des zweiten Teils gekoppelt ist.

3. Tragbare Peristaltikpumpe nach einem der obigen Ansprüche, bei der die Vorrichtungen zur Drehung des Rotors über einen externen Befehl mit einer Verlängerung (133) ausgestattet ist, die die Rotorenachse verlängert. An dieser befindet sich eine Bohrung (134), die es erlaubt, das Ende eines Werkzeugs mit entsprechender Passung aufzunehmen.

4. Tragbare Peristaltikpumpe nach einem der obigen Ansprüche, mit folgenden Merkmalen:
- Der Rotor und seine Rollen, die Antriebsvorrichtung und die Steuerungsvorrichtung bilden ein erstes Modul (100), das sogenannte Pumpenmodul.
- Das Stützstück gehört zu einem zweiten Modul (300), dem sogenannten Kassettenmodul.
- Die Module sind jeweils mit Vorrichtungen ausgestattet, die ihre Kopplung ermöglichen.
- Das Kassettenmodul enthält Vorrichtungen, um abwechselnd den Schlauch (202) aufzunehmen und ihn automatisch zu positionieren. Bei der Verkopplung der beiden Module wird der Schlauch gegen den abgerundeten Teil (308) gepresst und dort von den Rollen komprimiert. So bilden Schlauch und Tank ein drittes Modul (200), das sogenannte Tankmodul, das nach seinem Gebrauch einfach durch ein Anderes ersetzt werden kann.

5. Tragbare Peristaltikpumpe nach Anspruch 4, bei der das Kassettenmodul (300) mit einem Boden (305) ausgestattet ist, der wir eine Schublade in das Pumpenmodul greift. Dieser Boden hat eine U-förmige Aussparung (307), die so profiliert und bemessen ist, dass sie den Rotor umgreift. Seine abgerundete Sohle (308) stützt den Schlauch. Die Vorrichtungen zur Aufnahme und Positionierung des Schlauches enthalten einen ersten (313) und einen zweiten Kanal (314), die entlang den jeweiligen Seiten des Bodens verlaufen. Ein Ende der Kanäle ist nach außen zum Boden offen, während das andere, gegenüberliegende Ende jeweils in der Öffnung der Auslassung mündet.

6. Tragbare Peristaltikpumpe nach Anspruch 5, bei der die Kanäle Einrastvorrichtungen zur Fixierung des Schlauchs enthalten.

7. Tragbare Peristaltikpumpe nach Anspruch 6, bei der die Vorrichtungen zur Fixierung des Schlauches aus gekrümmten Anteilen (315, 316) der Kanäle bestehen. Diese sind so profiliert und bemessen, dass die gleichgeformten Teilstücke (204, 205) des Schlauches nicht verrutschen und über Einrasten festgehalten werden.

8. Tragbare Peristaltikpumpe nach einem der Ansprüche 4 bis 7, bei der das Kassettenmodul über Einrasten des Bodens in die Führung (102) des Pumpenmoduls in das Pumpenmodul montiert ist.

9. Tragbare Peristaltikpumpe nach einem der Ansprüche 4 bis 8, bei der das Kassettenmodul unter anderem mit einer Aussparung (301) zur Aufnahme des Flüssigkeitstanks ausgeführt ist.

10. Tragbare Peristaltikpumpe nach einem der obigen Ansprüche, bei der der Rotor mit Vorrichtungen ausgestattet ist, die erlauben, Positionsabstände zwischen der Rolle und dem Stützstück automatisch auszugleichen.

11. Tragbare Peristaltikpumpe nach Anspruch 10, bei der die Rolle (110) mit einem zylindrischen Gehäuse (127) und einer Achse (126) ausgestattet ist, auf der das Gehäuse drehbar montiert ist, so dass die Enden der Achse auf den länglichen Öffnungen (128) liegen, die radial im Rotor ausgerichtet sind. Die Ausgleichsvorrichtung enthält zwei Federn (129) auf Höhe der jeweiligen Enden der Rollenachse. Mit einem Mittelstück (130), das konzentrisch zum Rotor angebracht ist und einem gekrümmten Federarm (132), von dem ein Ende mit dem Mittelstück verbunden ist, während sich das andere Ende auf das entsprechende Ende der Rollenachse stützt. Diese wird so radial nach außen geschoben, wodurch die Rolle auf den Schlauch eine konstante Kompressionskraft ausübt.
